# EUROPEAN PATENT SPECIFICATION

(11) **EP 0 504 751 B1**
(45) Date of publication and mention of the grant of the patent: **14.06.2000**
(21) Application number: 92104355.0
(22) Date of filing: 13.03.1992
(51) Int. Cl.: C12N 15/24

(54) **Method for the preparation of interleukin 6**
Verfahren zur Herstellung von Interleukin-6
Méthode de préparation d'interleukine-6

(30) Priority: 22.03.1991 IT MI910774
(43) Date of publication of application: 23.09.1992
(73) Proprietor: CEINGE SOCIETA CONSORTILE a.r.l., 20131 Napoli (IT)
(72) Inventor: Ciliberto, Gennaro, I-20131 Napoli (IT); Arcone, Rosaria, I-20131 Napoli (IT)
(74) Representative: Minoja, Fabrizio, Dr.

(56) References cited:
- EP-A- 0 326 120
- EUROPEAN JOURNAL OF BIOCHEMISTRY vol. 198, no. 3, 15 June 1991, BERLIN;DE. pages 541 - 547; R. ARCONE ET AL: 'Single-step purification and structural characterization of human interleukin-6 produced in E.coli from a T7 RNA polymerase expreesion vector'
- MOLECULAR AND CELLULAR BIOLOGY vol. 10, no. 6, June 1990, WASHINGTON;US. pages 2443 - 2447; CLAY B. SIEGALL ET AL: 'Cell-specific toxicity of a chimeric protein composed of Interleukin-6 and Pseudomonas exotoxin_IL-6-PE40_ on tumor cells'
- JOURNAL OF MOLECULAR BIOLOGY vol. 189, no. 1, 5 May 1986, LONDON;GB. pages 113 - 130; F. W. STUDIER ET AL: 'Use of bacteriophage T7 RNA polymerase to direct selective high-level expression of cloned genes'
- JOURNAL OF BIOCHEMISTRY. vol. 104, no. 1, 1988, TOKYO JP pages 30 - 34; NAOTO TONOUCHI ET AL: 'High level expression of human BSF-2/IL-6 cDNA in E. coli using a new type of expression-preparation system'
- BIOTECHNOLOGY vol. 8, November 1990, NEW YORK US pages 1036 - 1040; HISASHI YASUEDA ET AL: 'High-level direct expression of semi-synthetic human interleukin-6 in E. coli and production of N-terminus met-free product'
- BIOTECHNOLOGY vol. 6, no. 7, July 1988, NEW YORK US pages 806 - 809; YOSHIHIRO ASAGOE ET AL: 'Human B-cell stimulatory factor-2 expressed in E.coli'

## Description

The present invention refers to a process for the preparation of interleukin 6 from transformed bacterial strains.

Interleukin 6 (IL-6/BSF-2) is a major cytokine which has been shown to play a central role in the physiology of several tissues and organs. The main targets of IL-6 are the immune system and the liver.

On B cells IL-6 induces the growth of hybridomas and plasmacytomas, the expression of class I antigens and the production and secretion of immunoglobulins. On hepatocytes IL-6 is a transcriptional inducer of a large group of plasmaproteins, including C-reactive protein, α₁-acid glycoprotein, haptoglobin, hemopexin, fibrinogen, etc., which are also known as acute phase proteins or reactants. IL-6, however, is also involved in the differentiation of T-cells and neurons. Besides the physiological activities of IL-6, deregulation of the expression of this cytokine has been postulated to be responsible for the development of several pathologies such as rheumatoid arthritis, cardiac mixoma and Castelman's disease, psoriasis, Kaposi sarcoma.

IL-6 is produced by a variety of cell types in response to interleukin-1 and tumor necrosis factor as a 26 kd glycoprotein. Glycosylation, however, is not essential for IL-6 activity because the protein produced in E. coli shows the same specific activity as natural IL-6 in biological assays on B-cells, hydribomas and hepatomas in culture.

Overproduction of functionally active IL-6 is of crucial importance for several reasons:
1) potentiation of the production of immunoglobulins by hydridomas in culture;
2) as a standard in the evaluation of blood and urine levels of IL-6 in several pathological conditions;
3) structural-functional studies;
4) development of structural agonists as possible therapeutical agents.

The therapeutic use of interleukin 6 has also been recently proposed (WO 9002570, WO 901943).

Strong efforts have been therefore paid for the development of a production process of interleukin 6 by recombinant DNA techniques.

Methods wherein, in order to increase stability in bacterial cells, the cytokine has been synthesized as a fusion protein either with the human interleukin 2 N-terminal peptide or with the human growth hormone have been respectively disclosed in J. Biochem. 104, 30-34, 1988 and in Biotechnology 6, 806-809, 1988.

These fusion have to be subjected to cleavage in vitro by specific endopeptidases and several purification steps are required in order to obtain the pure protein.

A similar method, based on the production of a fusion protein, has also been disclosed in WO 90/06370. A synthetic gene coding for interleukin 6 has been disclosed in GB-A-2217327. WO 88/00206 discloses a process for the preparation of recombinant IL-6 characterized by the secretion in the periplasma of the protein linked to a leader sequence which must be then removed during the purification.

The production of a IL-6 fusion protein in E. coli strains under the control of T7 promoter has been described in Mol. Cell. Biol. (1990) 6, 2443. The reported yield was however rather low (1 mg/ml), perhaps in view of the cumbersome extraction and purification method.

All the known processes suffer therefore from drawbacks due to difficulties in the isolation and purification of IL-6.

The process of the invention allows to overcome the problems of the prior art, allowing the production of IL-6 in very high yields and in an easily purifiable form.

The process of the invention comprises:
a) the culture of a bacterial strain E. coli BL 21 (DE3) Lys E transformed with the plasmid pT7.7/hIL-6 deposited at the ATCC under accession number 68507;
b) bacterial lysis, isolation by centrifugation of inclusion bodies containing interleukin-6 and their denaturation with guanidine hydrochloride;
c) dialysis of the denaturated proteins in two steps : a first one in the presence of 2Mm of reduced glutathione, and 0.2 mM oxidized glutathione against Tris/HCl pH 8.9, 1M guanidine hydrochloride and the second one against Tris/HCl pH 8.9 and 10% glycerol;
d) anion-exchange chromatography of the dialysate on DEAE-Sepharose CL-6B, eluting with a pH adjusted saline water-glycerol solution gradient, selecting and collecting the fractions containing interleukin-6 at the desired purity.

According to a preferred embodiment of the invention, the human interleukin 6 is overproduced in E. coli as a full length native protein without a leader peptide, in high yields (≥ 25 mg/ml) and after a single step of DEAE-Sepharose chromatography.

The use of expression systems using T7 bacteriophagus promoters is described, for instance, in EP-A-0178863 and in J. Mol. Biol. 189, 113-130, 1986.

The sequence of cDNA coding for IL-6 has already been described by several authors, cited for instance in WO 90/06370. As a source of cDNA coding for IL-6 the plasmid pUK-IL-6 disclosed in J. Immunol. 143, 1175-1182, 1989 was used in the present invention.

Escherichia coli strains carrying a gene coding for T7 RNA polymerase under control of an inducible promoter are known: the E. coli strain BL 21 (DE3) Lys E is described in Methods Enzymol 185, 60-89, (1990). In this strain, the gene coding for T7 RNA polymerase is under control of the inducible promoter lac UV5. It is known that the lac promoter of E. coli has a relatively low activity in the absence of lactose where as in the presence of an inducing agent such as lactose it self or isopropyl-β-D-thiogalactoside (IPTG) the promoter is de- repressed and causes an high level of transcription of the controlled gene.

According to the invention, the promoter obtainable from the plasmid pT7/7, disclosed in Proc. Natl. Acad. Sci. USA 82, 1074-1078 (1985) is used.

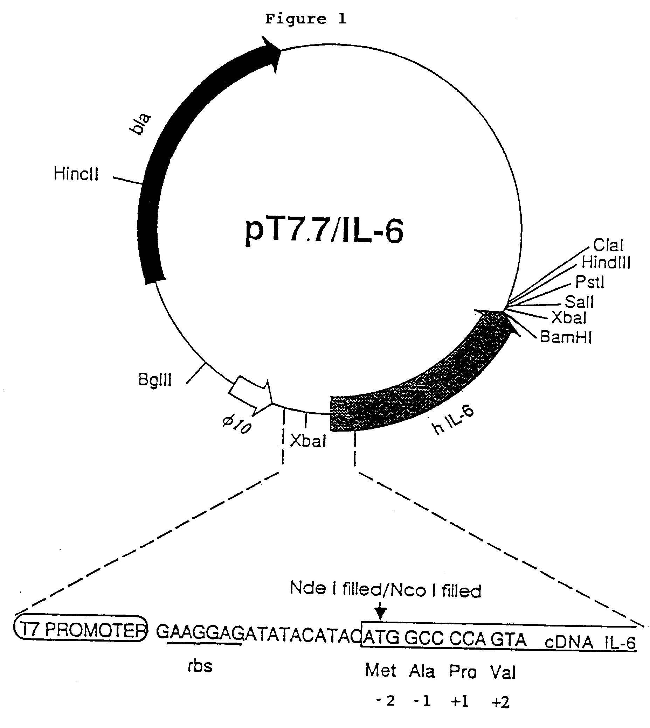

The plasmid obtained by cloning the Nco I (filled)-Bam HI fragment of pUK-IL-6 in the Nde I (filled)-Bam HI sites of pT7.7, named pT7.7/hIL-6 (figure 1) has been deposited at the ATCC under accession number 68507.

The dialysis of the denaturated extract is carried out in two steps: a first one, in the presence of GSH/GSSG against Tris/HCl pH 8.0, 1M guanidine hydrochloride and the second one against. Tris/HCl pH 8.9 and 10% glycerol. Step d) is preferably carried out using a NaCl 50 mM Tris/HCl, 10% glycerol solution pH 8.9 and the NaCl concentration of the gradient includes the range from approximately 0.0 to approximately 0.5 M.

In the following example, usual techniques were used, such as those disclosed in T. Maniatis et al., 1982, Molecular Cloning. For instance, commercial restriction enzymes or T4 DNA ligase were used according to the instructions provided by the suppliers.

The transformation was carried out by the calcium chloride method, disclosed in J. Mol. Biol. 53, 154, 1970.

### EXAMPLE

A Nco I (filled)-Bam HI purified fragment of 1,000 bp containing the coding region of mature hIL-6 plus 445 bp of the 3' untranslated sequence was excised from plasmid pUK-IL-6 and inserted in the pT7.7 expression vector digested with Nde I (filled)-Bam HI in the polylinker region downstream to the T7 promoter. The construct was controlled by restriction mapping and Sanger sequence analysis.

The resulting open reading frame encodes for two additional aminoacids, methionine (position-2) and alanine (position-1), with respect to the mature sequence of IL-6, the amino terminus of which corresponds to the proline in position+1 of our open reading frame. The plasmid pT7.7/hIL-6 was then introduced in E. coli, strain BL 21 (DE3) Lys E.

### IL-6 production in E. coli

A single colony was used to inoculate 5 ml L-broth containing 100 µg/ml ampicillin and grown overnight in shaken flasks at 37°C. The saturated culture was diluted 1:100 and when absorbance at 600 nm reached 1.0, isopropylthiogalactopyranoside (IPTG) was added to a final concentration of 0.4 mM and growth was continued for 3 hours.

The analysis on SDS/PAGE, using cells transformed with the plasmid vector as control and Coomassie staining of the gel showed a protein of the expected size (21 KDa), which was abundant in the IPTG induced bacteria and absent from control bacteria. Test carried out at different concentrations of IPTG (up to 1.2 mM) or with different induction times (1-6 hours) did not cause significant differences in the protein expression.

The identity of the 21 kDa protean was confirmed as IL-6 by Western blotting using a specific anti-IL-6 monoclonal antibody.

### Solubilization of IL-6 from inclusion bodies and refolding

After induction, cells were collected by centrifugation, resuspended in 50 mM Tris/HCl pH 7.5, 1 mM ethylendiamine tetracetic acid (EDTA), 1% Tween 20, 1 mM dithiothreitol (DTT), 1 mM phenylmethanesulfonyl fluoride (PMSF) and broken by 3 passes through French Press at 750 Psi (American Instrument Company). All the purification steps were performed at 4°C. The cell inclusion bodies were isolated from the cell lysate by centrifugation at 25,000 g for 30 min and then extracted stirring overnight in 6 M guanidine hydrochloride (Gdn-HCl) in Tris/HCl 50 mM pH 8.0 at a concentration of protein of 0.4 mg/ml. Extracted proteins were subjected to a first dialysis overnight against 50 mM Tris/HCl pH 8.9, 1 M Gdn-HCl, 2 mM reduced glutathione (GSH) and 0.2 mM oxidized glutathione (GSSG), followed by a further dialysis against 50 mM Tris/HCl pH 8.9, 10% glycerol.

### Protein determination, sodium dodecylsulphate-polycrylamide gel electrophoresis (SDS-PAGE) and immunostaining

The concentration of total protein at each step was determined by a Coomassie-blue G-250 binding assay as described in Anal. Biochem 72, 248-254, 1986 using the Bio-Rad Protein Assay and bovine serum albumin as a standard. 12% polyacrylamide SDS-PAGE electrophoresis gels were performed as described in Nature 227, 680-685, 1970.

Protein were stained by Coomassie Brilliant Blue R-250 and immunodetected following transfer to nitrocellulose filters (BA 85; Schleicher & Schuell) as described in Proc. Natl. Acad. Sci. USA 76, 4350-4354, 1979.

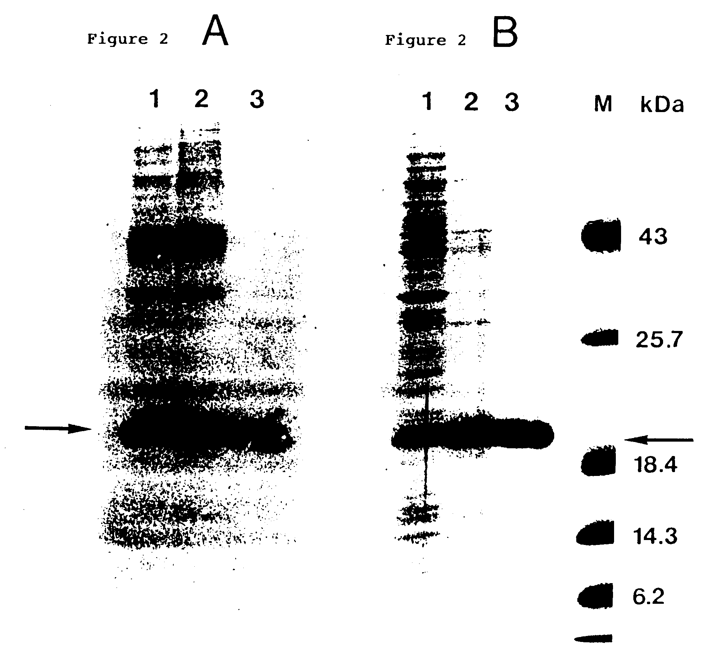

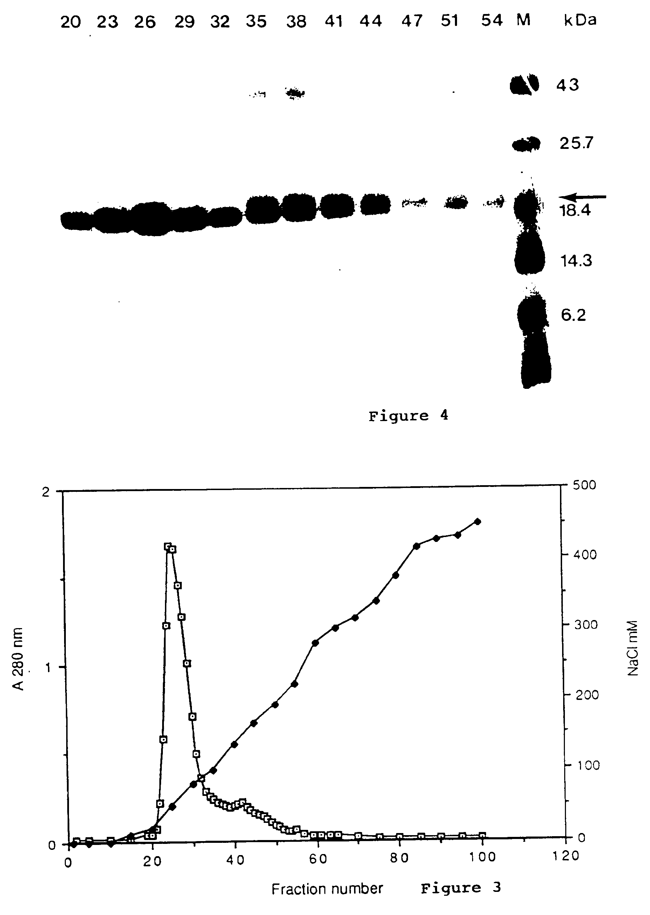

Figure 2a and 3b refer to the SDS/PAGE analysis in reducing conditions of bacterial proteins from BL 21 IPTG induced cells transformed with pT7.7/IL-6 plasmid. Arrow shows rhIL-6 protein.

Figure 2A concerns the immunoenzymatic Western blotting where as figure 2B concerns the Coomassie blue staining.
Lane 1: total proteins.
Lane 2: refolded proteins extracted from inclusion bodies
Lane 3: fraction number 26 from the DEAE column.

### Anionic exchange chromatography

A 1.5 x 8 cm column packed with DEAE-Sepharose CL-6B (Pharmacia) was equilibrated with 50 mM Tris/HCl pH 8.9, 10% glycerol. After loading of the crude refolded extract, the column was washed with 5 volumes of the same buffer and eluted with a linear gradient 0.0 to 0.5 M NaCl 50 mM Tris/HCl pH 8.9, 10% glycerol, collecting fractions of 2.2 ml volume each.

A large quantity of the leaded proteins elutes as a single and symmetric peak at 50 mM NaCl. IL-6 was found only in the fractions 20 to 35, highly purified from contaminating proteins, as it can be seen from SDS/PAGE analyses of the fractions 20-54. The figure 3 shows the elution pattern on DEAE-Sepharose Cl-6B. White squares line represents elution pattern monitored at 280 nm, the black squares line represent the linear gradient of NaCl. Figure 4 reports the SDS-PAGE analysis of the single fraction of the DEAE column. A single protein species may be visualized by the Coomassie staining of the gel, staining intensely with anti IL-6 specific antibody.

Table 1 summarizes the purification scheme. From 1 lt of bacterial culture, 3.8 gr wet weight bacteria were obtained. Extraction of inclusion bodies and renaturation gave rise to 29.2 mg of protein. The pure protein eluted from the DEAE column is about 25 mg.

**TABLE 1**

| Purification of recombinant human interleukin 6 from E. coli 1 litter culture (3.8 gr bacteria wet weight). | | |
|---|---|---|
| | Total protein (mg) | Yield (%) |
| Inclusion bodies | 44.4 | |
| Refolded extract | 29.2 | 65.7 |
| rhIL-6 from DEAE column (pooled fractions) | 25.0 | 85.6 |

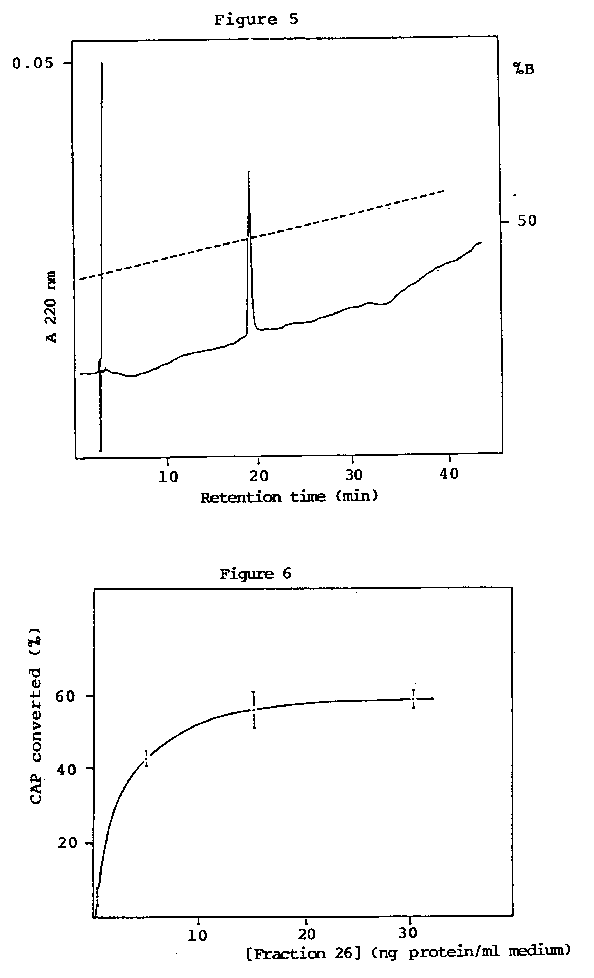

### High pressure liquid chromatography (HPLC) analysis (Fig. 5)

The homogeneity of recombinant IL-6 preparation was tested by reverse-phase HPLC on a Vydac C4 column (5 µm); the elution system was trifluoroacetic acid (TFA) 0.1% (solvent A) and TFA 0.07% in acetonitrile (solvent B). A portion (4 µg) of the DEAE fractions were diluted to 100 µl with TFA 0.1% and loaded onto the column equilibrated with 40% B. The column was washed for 5 min and the protein was eluted with a linear gradient from 40% to 60% B over 40 min at a flow rate of 1 ml/min. rhIL-6 eluted as a single peak having a retention time of 19 minutes.

### Titration of SH groups

The free SH groups content of recombinant IL-6 was determined by titration with 1 mM 5,5'-dithio-bis-nitrobenzoic acid (NBs₂) in 0.1 M Tris-HCl, pH 8.0 according to Arch. Biochem. Biophys. 82, 70-75, 1959. The reaction was carried out with 0.1-0.2 mg of protein both in the presence and in the absence of 0.4% SDS by monitoring the increasing absorbance at 412 nm following the addition of the protein solution.

The analysis evidenced the absence of free SH groups.

### Electrospray mass spectrometric analysis

A freeze dried sample of HPLC purified rhIL-6 was dissolved in 35 µl of a 1:1 H₂O/methanol solution containing 3% acetic acid to a final concentration of IL-6 of about 30 pmol/µl. Electrospray mass spectrometric analysis was carried out by injecting 10 µl of this solution into a VG BIO Q mass spectrometer (VG Analytical) consisting of an electrospray ion source followed by a quadrupole mass analyzer, at a flow rate of 2 µl/min.

The mass spectrometer was scanned from m/z 800-1600 in 20 s at a mass resolution of unity. Data were acquired by summing several scans to obtain the final spectrum; each molecular species produced a series of multiply charged ions from which the molecular weight was determined by simple data system routines. Mass scale calibration employed the multiply charged ions from a separate introduction of myoglobin (average molecular weight = 16,950.6).

Surprisingly, two molecular species could be distinguished in the protein sample which according to the analysis gave origin to a single symmetric peak. The major component had a calculated average molecular mass of 20,905.78 which agrees very well with that predicted on the basis of the natural IL-6 amino acid sequence in the oxidized form (20,905.30). The minor component, which accounts for less than 25% of the total protein sample, displayed an average molecular weight of 21,108.08; on the basis of the accurate mass measurement and of the genetic construct, this component was identified as the N-terminal Met-Ala extended form of IL-6 (Met-Ala IL-6) for which a theoretical molecular mass of 21,108.57 was predicted.

### Protein sequencing

Automated Edman degradation of rhIL-6 was carried out on a Chelsea Instrument CI 4000 gas phase protein sequencer. 1 nmol of protein was loaded onto the glass fibre disk and the sequence run was performed according to manufacturer's instructions. Phenylthioidantoin (PTH) amino acids were identified on a Beckman System Gold HPLC apparatus according to J. Chromatog. 270, 371-377, 1983.

The presence of 2 sequences was evidenced also from this analysis, the mayor one corresponding to the natural protein and the minor one having a dipeptide Met-Ala extension at the aminoterminus.

### Peptide mapping

rhIL-6 (1 mg) was digested with trypsin (TPCK-treated, Sigma) in 0.4% ammonium bicarbonate pH 8.5 at 37°C for 6 hours with an enzyme to substrate ratio of 1:50 (w:w). The sample was lyophilized, dissolved in 0.1% TFA and 1/20th was analyzed by Fast Atom Bombardment mass spectrometry (FAB/MS) using a VG ZAB 2SE double focusing mass spectrometer (VG Analytical) fitted with a caesium gun operating at 25 kv (2 µA). The sample was loaded onto a glycerol-thioglycerol probe tip; spectra were recorded on UV-sensitive paper and manually counted.

All the signals were assigned to the corresponding peptides along the IL-6 sequence with the exception of the signal at m/z 1774. This signal occurred 2 mass units lower than the expected mass values for the fully reduced peptide 71-86, and it was interpreted as originated from the presence of an intramolecular S-S bridge linking Cys 75-Cys 83. Due to the absence of any free SH group in the protein, the remaining two Cys residues at positions 44 and 50 must be involved in the second disulphide bond. This S-S bridge was indeed identified in the FAB analysis of a peptic and tryptic double digest of rhIL-6.

Indeed, the disulphide structures of rhIL-6 were found to be essential for maintaining the biological activity of the protein, as site-directed mutagenesis of any of the four Cys residues resulted in a polypeptide chain lacking any detectable activity (Arch. Biochem. Biophys. 268, 81-92, 1989).

The remaining peptide mixture was fractionated by HPLC on a µBondapack C₁₈ column (Waters/Millipore) using 0.1% TFA as solvent A and 0.07% TFA in acetonitrile as solvent B. Peptides were eluted by a linear gradient from 5% to 60% B in 70 min. Fractions were manually collected, dried down in a Speedy Vac (Savant) centrifuge and analyzed by FAB/MS. In all cases, peptides were located within the human IL-6 sequence on the basis of their molecular weight, using a suitable computer programme.

It is therefore possible to conclude that the obtained rIL-6 is identical to the known IL-6, about 20% of the product being in the extended form Met-Ala-IL-6.

### IL-6 bioassay in hepatoma cells

IL-6 assays in Human Hepatoma Hep 3B cells were performed according to the method disclosed in EMBO J. 8, 3773-3779, 1989. Plasmid 3'Δ-121/-46 CRP-CAT (Nucl. Acid Res. 16, 3195-3207, 1988) carrying a fusion between a segment of C-reactive protean (CRP) promoter linked to the SV40 early promoter and to the bacterial chloramphenicol acetyltransferase gene (CAT) was transfected in the cells using the calcium phosphate precipitation technique (Virology 52, 456, 1973). After 15 h, precipitates were washed off and the cells were treated with fraction 26 of the DEAE-Sepharose CL 6B column. Induction was carried out for 24 h; cell extracts and chloramphenicolacetyltransferase assays were performed as described (DNA Cloning: A Practical Approach (Glover, M. D., ed), pag. 143-190, IRL Press, Oxford) using 20 µg cell extracts incubated at 37°C for 15 min. After autoradiography, individual spots were cut, eluted and the percentage of acetylated versus [¹⁴C]chloramphenicol was determined (CAP percent conversion).

The result reported in Figure 6 show that the activity of rIL-6 was 1.5-2 x 10⁸ U/mg of protein.

The purified E. coli preparation was also tested for its ability to support the growth of the murine IL-6 dependent B cell hybridoma 7TDl. The results (Table 2) show that E. coli rhIL-6 has a specific activity of 1.5 x 10⁸ U/mg.

**TABLE 2**

| Hybridoma growth factor activity of rhIL-6 | | |
|---|---|---|
| | U/ml | U/mg |
| Fraction 26 (6.3 mg/ml) | 9.5 x 10⁸ | 1.5 x 10⁸ |

## Claims

1. A process for the preparation of interleukin 6 from transformed bacterial strains which comprises:
a) the culture of a bacterial strain E. coli BL 21 (DES) Lys E transformed with the plasmid pT7.7/hIL-6 deposited at the ATCC under accession number 68507;
b) bacterial lysis, isolation by centrifugation of inclusion bodies containing interleukin-6 and their denaturation with guanidine hydrochloride;
c) dialysis of the denaturated proteins in two steps: a first one in the presence of a 2mM of reduced glutathione, and 0.2 mM oxidized glutathione against Tris/HCL pH 8.9, 1M guanidine hydrochloride and the second one against Tris/HCL pH 8.9 and 10% glycerol.
d) anion-exchange chromatography of the dialysate on DEAE-Sepharose CL-6B, eluting with a pH adjusted saline water-glycerol solution gradient, selecting and collecting the fractions containing interleukin-6 at the desired purity.

2. A process according to claim 1 wherein the eluent of the DEAE-Sepharose is NaCl 50 mM Tris/HCl, 10% glycerol solution pH 8.9 and the NaCl concentration of the gradient includes the range from approximately 0.0 to approximately 0.5 M.

## Patentansprüche

1. Verfahren zur Herstellung von Interleukin-6 aus transformierten Bakterienstämmen, umfassend:
a) Kultivierung des Bakterienstammes E. coli BL 21 (DE3) Lys E, transformiert mit dem Plasmid pT7.7/hIL-6, hinterlegt bei der ATCC unter der Hinterlegungsnummer 68507;
b) Lyse der Bakterien, Isolation der Einschlußkörperchen, die Interleukin-6 enthalten, durch Zentrifugation und deren Denaturierung mit Guanidiniumhydrochlorid;
c) Dialyse der denaturierten Proteine in zwei Stufen: einer ersten in Gegenwart von 2 mM reduziertem Glutathion und 0,2 mM oxidiertem Glutathion gegen Tris/HCl pH 8,9, 1M Guanidiniumhydrochlorid und einer zweiten gegen Tris/HCl pH 8,9 und 10% Glycerin;
d) Anionenaustauschchromatographie des Dialysats an DEAE-Sepharose CL-6B, Elution mit einem Salzlösungs-Glycerinlösungs-Gradienten mit eingestelltem pH-Wert, Auswählen und Sammeln der Fraktionen, die Interleukin-6 in der gewünschten Reinheit enthalten.

2. Verfahren nach Anspruch 1, wobei das DEAE-Sepharose-Elutionsmittel NaCl 50 mM Tris/HCl, 10% Glycerinlösung pH 8,0 ist und die NaCl-Konzentration des Gradienten den Bereich von etwa 0,0 bis etwa 0,5 M umfaßt.

## Revendications

1. Procédé pour la préparation d'interleukine 6 à partir de souches bactériennes transformées qui comprend :
a) la culture d'une souche bactérienne E.coli BL 21 (DE3) Lys E transformée avec le plasmide pT7.7/hIL-6 déposée à l'ATCC sous le numéro d'admission 68507 ;
b) une lyse bactérienne, un isolement par centrifugation de corps d'inclusion contenant de l'interleukine 6 et leur dénaturation par le chlorhydrate de guanidine ;
c) la dialyse des protéines dénaturées en deux étapes : la première en présence de 2mM de glutathione réduite, et 0,2 mM de glutathione oxydée contre du Tris/HCl pH 8,9, 1M de chlorhydrate de guanidine et la seconde contre du Tris/HCl pH 8,9 et du glycérol 10% ;
d) une chromatographie échangeuse d'anions du dialysat sur DEAE-Sépharose CL-6B, en éluant avec un gradient de solution eau saline -glycérol au pH ajusté, sélectionnant et collectant les fractions contenant l'interleukine 6 à la pureté désirée.

2. Procédé selon la revendication 1 dans lequel l'éluant pour le DEAE-Sépharose est une solution de NaCl 50 mM Tris/HCl, glycérol 10%, pH 8,9 et la concentration en NaCl du gradient est dans la plage d'environ 0,0 à environ 0,5M.
